# EUROPEAN PATENT APPLICATION

(11) **EP 3 306 318 A1**
(43) Date of publication of application: **11.04.2018**
(21) Application number: 16803314.0
(22) Date of filing: 30.05.2016
(51) Int. Cl.: G01N 33/543, G01N 5/02, G01N 35/00

(54) **DETECTION METHOD AND DETECTION DEVICE**

(30) Priority: 29.05.2015 JP 2015110302
(71) Applicant: Kyocera Corporation, Kyoto-shi, Kyoto 612-8501 (JP)
(72) Inventor: TANAKA, Hiroyasu, Kyoto-shi Kyoto 612-8501 (JP); KATTA, Hiroshi, Kyoto-shi Kyoto 612-8501 (JP)
(74) Representative: Viering, Jentschura & Partner mbB Patent- und Rechtsanwälte
(86) International application number: PCT/JP2016/065941
(87) International publication number: WO 2016/194879

(57) **Abstract**

A detection method of a detection target contained in a sample is provided. In addition, a detection device for detecting a detection target contained in a sample. A detection method includes a first detection step of acquiring a detection value from a first signal value measured in a first measurement step and a second signal value measured in a second measurement step. A detection device includes a first detecting section which acquires a detection value from a first signal value measured in a first measurement section and a second signal value measured in a second measurement section.

## Description

### Technical Field

The invention relates to a detection method and a detection device of a detection target contained in a sample.

### Background Art

A method of detecting a target substance in a sample by using a biosensor which includes a detecting element, to a surface of which an antibody binds, has been known (for example, see Patent Literature 1 or 2).

### Citation List

### Patent Literature

Patent Literature 1: Japanese Patent Publication No. JP-B2 2625577
Patent Literature 2: Japanese Unexamined Patent Publication JP-A 2001-13142

### Summary of Invention

### Technical Problem

However, due to an influence of a substance other than a detection target contained in the sample, there is a case where the conventional detection method and the conventional detection device cannot accurately detect the detection target in the sample. In addition, due to influences of viscosity, density, and the like, for example, there is a case where the conventional detection method and the conventional detection device cannot accurately detect a signal value of the detection target.

For this reason, a detection method and a detection device have been requested which can further accurately detect the detection target in the sample by reducing the influence of the substance other than the detection target contained in the sample as well as influences of a difference in viscosity and a difference in density among the samples.

### Solution to Problem

A detection method according to an embodiment of the invention is a detection method of a detection target contained in a sample, including: a preparation step of preparing a primary substance which binds to a surface of a detection body and reacts with the detection target; a first reaction step of supplying the sample to the surface of the detection body and forming a primary reactant on the surface of the detection body through a reaction of the detection target with the primary substance; a first supply step of supplying a first liquid to the surface of the detection body after the first reaction step; a first measurement step of measuring a first signal value after the first supply step, the first signal value being based on a surface state of the detection body; a signal amplification step of supplying a signal amplification substance to the surface of the detection body and changing the surface state of the detection body through a reaction in which the primary reactant formed in the first reaction step is involved, after the first measurement step; a second measurement step of measuring a second signal value after the signal amplification step, the second signal value being based on the surface state of the detection body; and a first detection step of acquiring a detection value from the first signal value and the second signal value.

A detection device according to an embodiment of the invention is a detection device which detects a detection target contained in a sample, including: a first reaction section which supplies the sample to a primary substance and forms a primary reactant on a surface of a detection body through a reaction of the detection target with the primary substance, the primary substance binding to the surface of the detection body and reacting with the detection target; a first supply section which supplies a first liquid to the surface of the detection body; a first measurement section which measures a first signal value which is based on a surface state of the detection body, after the first supply section supplies the first liquid to the surface of the detection body; a signal amplification section which supplies a signal amplification substance to the surface of the detection body and changes the surface state of the detection body through a reaction in which the primary reactant is involved; a second measurement section which measures a second signal value which is based on the surface state of the detection body to which the signal amplification substance has been supplied; a first detecting section which acquires a detection value from the first signal value and the second signal value; and a display section which displays the detection value acquired by the first detecting section.

### Advantageous Effects of Invention

According to the detection method and the detection device according to the embodiment of the invention, an influence of a substance other than the detection target which is contained in the sample can be reduced by having a configuration as described above. Therefore, the detection target which is contained in the sample can further accurately be detected. In addition, influences of viscosity and density caused by the sample can be reduced. Therefore, the detection target which is contained in the sample can be further accurately detected.

### Brief Description of Drawings

FIG. 1 is a view illustrating a flowchart of a detection method according to an embodiment of the invention;
FIG. 2 is a view illustrating a flowchart of a detection method according to an embodiment of the invention;
FIG. 3 is a block diagram illustrating a detection device according to an embodiment of the invention;
FIG. 4 is a perspective view of a biosensor device 200 according to an embodiment of the invention;
FIG. 5 is an exploded perspective view of the biosensor device 200 according to the embodiment of the invention;
FIG. 6 is a plan view of a detecting element 3 according to an embodiment of the invention;
FIG. 7 is a schematic graph of a signal value acquired by the detection method according to the embodiment of the invention; and
FIG. 8 is a graph of experiment data relating to the detection method according to the embodiment of the invention.

### Description of Embodiments

### <Detection Method>

### (First Embodiment)

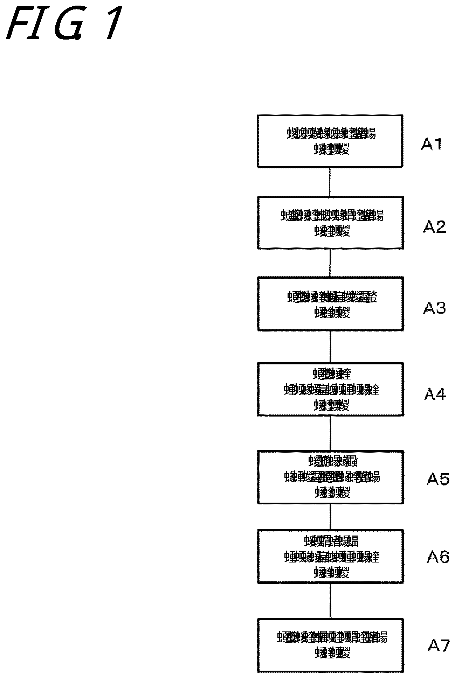

FIG. 1 is a flowchart of a detection method according to a first embodiment of the invention.

The detection method according to the first embodiment of the invention is a detection method of a detection target contained in a sample, including:
a preparation step A1 of preparing a primary substance which binds to a surface of a detection body and reacts with a detection target;
a first reaction step A2 of supplying the sample to the surface of the detection body and forming a primary reactant on the surface of the detection body through a reaction of the detection target with the primary substance;
a first supply step A3 of supplying a first liquid to the surface of the detection body after the first reaction step A2;
a first measurement step A4 of measuring a first signal value after the first supply step A3, the first signal value being based on a surface state of the detection body;
a signal amplification step A5 of supplying a signal amplification substance to the surface of the detection body and changing the surface state of the detection body through a reaction in which the primary reactant formed in the first reaction step A2 is involved, after the first measurement step A4;
a second measurement step A6 of measuring a second signal value after the signal amplification step A5, the second signal value being based on the surface state of the detection body; and
a first detection step A7 of acquiring a detection value from the first signal value and the second signal value.

The preparation step A1 is a step of preparing the primary substance which binds to the surface of the detection body and reacts with the detection target.

The first reaction step A2 is performed by using: the detection body to which the primary substance binds; a supply channel through which the sample is supplied to this detection body; a pump which causes a flow of the sample through the supply channel; and the like. However, a configuration thereof is not limited.

The first supply step A3 is performed by using a supply channel through which the first liquid is supplied, a pump, and the like. However, a configuration thereof is not limited, and the first supply step A3 may be performed in a similar manner to the first reaction step A2.

The first measurement step A4 may be performed by using a device or the like which includes an element which inputs a signal to the detection body and which acquires a prescribed signal value on the basis of a signal outputted from the detection body. However, a configuration thereof is not limited.

The signal amplification step A5 is performed by using the detection body in the first reaction step A2, a supply channel through which the signal amplification substance is supplied to this detection body, a pump, and the like. However, a configuration thereof is not limited, and the signal amplification step A5 may be performed by the reaction section 20.

The second measurement step A6 may be performed by using a device or the like which includes the element which inputs the signal to the detection body and which acquires a prescribed signal value on the basis of the signal outputted from the detection body. However, a configuration thereof is not limited, and the second measurement step A6 may be performed in a similar manner to the first measurement step A4.

The first detection step A7 may be performed by using an arithmetic unit or the like which includes an arithmetic element which acquires the detection value from the first signal value and the second signal value. However, a configuration thereof is not limited.

Here, in this specification, the "sample" may be a biological sample as is such as blood, urine, saliva, or phlegm, or may be a biological sample which is diluted by a buffer solution or the like. Note that the "sample" may be a sample other than the biological sample.

Here, in the case where the same amount of the detection target is contained in the plural samples, and such a detection target is detected, even when the first liquid is supplied to the surface of the detection body in the first supply step A3, a foreign substance which is contained in each of the samples and is a different substance from the detection target, cannot possibly be removed completely from the surface of the detection body. Thus, the signal values for the detection targets possibly differ from each other. However, according to the detection method of this embodiment, the detection value is acquired from the first signal value and the second signal value, and thus it is possible to reduce an influence of the foreign substance or influences of a difference in viscosity and a difference in density among the samples. This is because the detection value acquired from the first signal value and the second signal value is not influenced by an amount of the foreign substance remaining on the surface of the detection body. Just as described, when the influence of the foreign substance (a residue) among the samples is reduced, it is possible to further accurately detect the detection target contained in each of the samples.

Hereinafter, a description will sequentially be made on the detection method in this embodiment.

In the preparation step A1 in this embodiment, the primary substance which binds to the surface of the detection body and reacts with the detection target is prepared.

In this specification, examples of the "detection target" are an antigen and an antibody; however, the "detection target" is not limited thereto. Hereinafter, the detection target will be also described as the antigen.

In this specification, examples of the "detection body" are elements which output a signal value, such as a surface acoustic wave element, QCM (Quartz Crystal Microbalance), SPR (Surface Plasmon Resonance), and a FET (Field Effect Transistor). However, the "detection body" is not limited thereto.

In the first reaction step A2 in this embodiment, the sample is supplied to the surface of the detection body, and the primary reactant is formed on the surface of the detection body through the reaction of the detection target with the primary substance.

In this specification, the "primary substance" is not particularly limited as long as the "primary substance" is a substance which specifically reacts with the detection target. Examples of the "primary substance" include: an antibody which binds to an antigen when the detection target is said antigen; and an antigen which binds to an antibody when the detection target is said antibody.

In this specification, the "primary reactant" means: a capturing body which is acquired when the primary substance captures the detection target; a composite body in which the detection target binds to the primary substance; a dissociated body which is acquired when a part of the primary substance binds to the detection target and the part is dissociated from the primary substance; and the like, for example. Although not limited thereto, the "primary reactant" includes a composite body which is acquired through the reaction of the antigen with the antibody, and the like.

Note that, as a modified example, as described above, in the first reaction step A2, the primary reactant can be also formed when the detection target binds to the part of the primary substance and the part is dissociated from the primary substance.

In the first supply step A3 in this embodiment, the first liquid is supplied to the surface of the detection body.

In this specification, the "first liquid" may be a buffer solution or the like, for example. Examples of the buffer solution include a phosphate buffer solution, a citrate buffer solution, a boric acid buffer solution, a HEPES (4-(2-hydroxyethyl)-1-piperidineethanesulfonic acid) buffer solution, a tris(hydroxymethyl)aminomethane buffer solution, and a MOPS (3-morpholinopropanesulfonic acid) buffer solution. However, the buffer solution is not limited thereto, and a well-known buffer solution may appropriately be used. In addition, the buffer solution may contain sodium chloride, potassium chloride, magnesium chloride, zinc chloride, or EDTA (ethylenediaminetetraacetic acid), and may further contain a surfactant such as Tween 20 (registered trademark), Triton X-100 (registered trademark), or Brij 35 (registered trademark) when necessary. Furthermore, a blocking substance may be mixed in the buffer solution when necessary. As the blocking substance, BSA (bovine serum albumin), casein, polyethylene glycol, an MPC (methacryloyloxyethyl phosphorylcholine) polymer, a betaine polymer, and a HEMA (hydroxyethylmethacrylate) polymer are exemplified. These points also apply to a second liquid and a third liquid, which will be described below.

In the first measurement step A4 in this embodiment, the signal value based on the surface state of the detection body is measured.

In this embodiment, the surface acoustic wave element may be formed in the surface of the detection body, and a phase characteristic value of the surface acoustic wave element may be used as the signal value based on the surface state of the detection body.

In addition, in this embodiment, the signal value based on the surface state of the detection body may be a value measured by a method selected from a QCM (Quartz Crystal Microbalance) sensor, a SPR (Surface Plasmon Resonance) sensor, and a FET (Field Effect Transistor) sensor.

In the signal amplification step A5 in this embodiment, the signal amplification substance is supplied to the surface of the detection body, and the surface state of the detection body is changed through the reaction in which the primary reactant formed in the first reaction step A2 is involved.

In this specification, the "signal amplification substance" is not particularly limited as long as the "signal amplification substance" is a substance which changes the surface state of the detection body. Examples of the "signal amplification substance" include a labeled secondary antibody, labeled peptide, labeled ligand, and labeled aptamer, each of which specifically reacts with the primary reactant. A label is not particularly limited as long as the label changes the surface state of the detection body. Examples of the label include protein such as streptavidin, biotin, an enzyme, a phosphor, and a nanoparticle such as a metallic particle.

In this specification, the "reaction in which the primary reactant formed in the first reaction step is involved" is not particularly limited as long as it is a reaction which changes the surface state of the detection body in accordance with an amount of the primary reactant. Examples of the "reaction in which the primary reactant formed in the first reaction step is involved" include a binding reaction of the primary reactant formed in the first reaction step A2 with the signal amplification substance, an enzymatic reaction of the primary reactant formed in the first reaction step A2 with the signal amplification substance, and a reductive reaction of the primary reactant formed in the first reaction step A2 with the signal amplification substance. The primary substance may directly react with the signal amplification substance, or the primary substance may indirectly react with the signal amplification substance via another substance.

In this embodiment, between the first measurement step A4 and the signal amplification step A5, the detection method may further comprise an additional reaction step of supplying at least one additional reaction substance to the surface of the detection body. In addition, the at least one additional reaction substance may have a plurality of additional reaction substances, and the plurality of additional reaction substances may be supplied one by one.

In the case where the additional reaction step is performed, the signal amplification substance may not specifically react with the primary reactant. Examples of the signal amplification substance may include: substrates such as streptavidin, 3,3'-diaminobenzidine, 3-amino-9-ethylcarbazole, 4-chloro-1-naphthol, and 5-bromo-4-chloro-3-indolylphosphate/nitro blue tetrazolium; and combinations of metallic ions and a reducing agent such as a combination of chloroauric acid and hydroxylamine hydrochloride and a combination of silver nitrate and iron sulfate.

In this specification, examples of the "additional reaction substance" include: biotin; streptavidin; enzyme-labeled antibodies such as a biotin-labeled antibody, a peroxidase-labeled antibody, and an alkaline phosphatase-labeled antibody; a nanoparticle-labeled antibody such as a metallic particle-labeled antibody; enzyme-labeled streptavidin such as peroxidase-labeled streptavidin and alkaline phosphatase-labeled streptavidin; and nanoparticle-labeled streptavidin such as metallic particle-labeled streptavidin.

The additional reaction substance should be selected in accordance with the selected signal amplification substance. For example, in the case where the signal amplification substance is streptavidin, the additional reaction substance is the biotin-labeled antibody. For example, in the case where the signal amplification substance is 3,3'-diaminobenzidine, the additional reaction substances are a biotin-labeled secondary antibody and peroxidase-labeled streptavidin. For example, in the case where the signal amplification substance is 5-bromo-4-chloro-3-indolylphosphate/nitro blue tetrazolium, the additional reaction substances are the biotin-labeled secondary antibody and alkaline phosphatase-labeled streptavidin. For example, in the case where the signal amplification substances are chloroauric acid and hydroxylamine hydrochloride, the additional reaction substances are the biotin-labeled secondary antibody and Au particle-labeled streptavidin.

In the additional reaction step, the additional reaction substance may repeatedly be supplied to further amplify the change in the surface state of the detection body. For example, in the case where the signal amplification substance is streptavidin and the additional reaction substance is the biotin-labeled antibody, the biotin-labeled secondary antibody is supplied firstly, streptavidin is supplied secondly, the biotin-labeled antibody is supplied thirdly, streptavidin is supplied fourthly, and the biotin-labeled antibody is supplied fifthly in the additional reaction step, and then streptavidin is supplied in the signal amplification step A5.

In this embodiment, between the first reaction step A2 and the first supply step A3, the detection method may further comprise a precursor reaction step of supplying at least one precursor reaction substance to the surface of the detection body. In addition, the at least one precursor reaction substance may have a plurality of precursor reaction substances, and the plurality of precursor reaction substances may be supplied one by one.

In the case where the precursor reaction step is performed, the signal amplification substance may not specifically react with the primary reactant. Examples of the signal amplification substance may include: the substrates such as streptavidin, 3,3'-diaminobenzidine, 3-amino-9-ethylcarbazole, 4-chloro-1-naphthol, and 5-bromo-4-chloro-3-indolylphosphate/nitro blue tetrazolium; the combinations of the metallic ions and the reducing agent such as the combination of chloroauric acid and hydroxylamine hydrochloride and the combination of silver nitrate and iron sulfate; and the like.

In this specification, examples of the "precursor reaction substance" include: biotin; streptavidin; an enzyme-labeled antibodies such as a biotin-labeled antibody, a peroxidase-labeled antibody, and an alkaline phosphatase-labeled antibody; a nanoparticle-labeled antibody such as a metallic particle-labeled antibody; enzyme-labeled streptavidin such as peroxidase-labeled streptavidin and alkaline phosphatase-labeled streptavidin; and nanoparticle-labeled streptavidin such as metallic particle-labeled streptavidin.

The precursor reaction substance should be selected in accordance with the selected signal amplification substance. For example, in the case where the signal amplification substance is streptavidin, the precursor reaction substance is the biotin-labeled antibody. For example, in the case where the signal amplification substance is 3,3'-diaminobenzidine, the precursor reaction substances are the biotin-labeled secondary antibody and peroxidase-labeled streptavidin. For example, in the case where the signal amplification substance is 5-bromo-4-chloro-3-indolylphosphate/nitro blue tetrazolium, the additional reaction substances are the biotin-labeled secondary antibody and alkaline phosphatase-labeled streptavidin. For example, in the case where the signal amplification substances are chloroauric acid and hydroxylamine hydrochloride, the precursor reaction substances are the biotin-labeled secondary antibody and Au particle-labeled streptavidin.

In the precursor reaction step, the precursor reaction substance may repeatedly be supplied to further amplify the change in the surface state of the detection body. For example, in the case where the signal amplification substance is streptavidin and the precursor reaction substance is the biotin-labeled antibody, the biotin-labeled secondary antibody is supplied firstly, streptavidin is supplied secondly, the biotin-labeled antibody is supplied thirdly, streptavidin is supplied fourthly, and the biotin-labeled antibody is supplied fifthly in the precursor reaction step. Then, after the first supply step A3 and the first measurement step A2, streptavidin is supplied in the signal amplification step A5.

Note that the signal amplification substance, the additional reaction substance, and the precursor reaction substance may be diluted by the buffer solution or the like.

Note that, in the signal amplification step A5, the signal amplification substance can also bind to the detection target in the primary reactant.

Note that, in this embodiment, after the signal amplification step A5 and before the second measurement step A6, the detection method may further comprise a second supply step of supplying the second liquid to the surface of the detection body.

According to this, the signal amplification substance which has not reacted with the primary reactant in the signal amplification step A5 is removed from the surface of the detection body. In this way, it is possible to reduce an influence of the signal amplification substance which has not reacted with the primary reactant on the second signal value, and it is possible to improve accuracy of the signal value which is related to the detection target and is measured in the second measurement step A6.

Note that, in this specification, the "second liquid" may be the buffer solution or the like, for example.

In addition, in this embodiment, the first liquid and the second liquid can be the same type of the liquid.

In the second measurement step A6 in this embodiment, the signal value based on the surface state of the detection body is measured.

This step only has to be performed in a similar manner to the above-described first measurement step A4.

In the first detection step A7 in this embodiment, the detection value is acquired from the first signal value and the second signal value.

In this embodiment, the second signal value may be larger than the first signal value. Here, a magnitude of the signal value may be determined from an absolute value of a difference from the signal value before the first reaction step A2, for example.

Note that, in this embodiment, the sample which further includes the foreign substance composed of a different substance from the detection target may be used.

In this case, the foreign substance which differs from the detection target is adhered to the surface of the detection body in the first reaction step A2. Thereafter, the first liquid is supplied to the surface of the detection body in the first supply step A3. As a result, there is a case where the foreign substance cannot be removed completely from the surface of the detection body and remains thereon. Therefore, the first signal value is influenced by the foreign substance which differs from the detection target and which remains on the surface of the detection body.

In such a case, in order to reduce the influence of the residue of this foreign substance, the detection value is acquired from the first signal value and the second signal value. In this way, it is possible to remove the signal value which is derived from the foreign substance remaining on the surface of the detection body, from the second signal value, and therefore it is possible to measure the further accurate signal value of the detection target.

In an embodiment of the invention, a second reaction step may be provided as an aspect of the signal amplification step A5. In the second reaction step, a secondary substance which reacts with the primary reactant is supplied to the surface of the detection body, and a secondary reactant is formed on the surface of the detection body through a reaction of the primary reactant with the secondary substance.

The second reaction step is performed by using the detection body in the first reaction step, a supply channel through which the secondary substance is supplied to this detection body, a pump, and the like. However, a configuration thereof is not limited, and the second reaction step may be performed in a similar manner to the first reaction step.

In this specification, the "secondary substance" is not particularly limited as long as the "secondary substance" is a substance which specifically reacts with the primary reactant. For example, the "secondary substance" includes a secondary antibody and the like, and may be an aspect of the signal amplification substance.

Note that, as the secondary substance, the labeled secondary antibody which is labeled with biotin, the enzyme, the nanoparticle, the metallic nanoparticle, or the like can be used, for example. By using the labeled secondary antibody which is prepared by labeling the secondary substance, it is possible to amplify the signal value which is derived from the secondary substance, and therefore it is possible to detect the detection target with higher sensitivity.

In this specification, the "secondary reactant" means: a capturing body which is acquired when the primary reactant captures the secondary substance; a composite body of the primary reactant and the secondary substance; and the like, for example. Although not limited thereto, the "secondary reactant" includes: a composite body of the antigen, a primary antibody, and the secondary antibody; and the like.

Note that molecular weight of the secondary reactant may be higher than molecular weight of the primary reactant. According to this, the detection target can be detected with the high sensitivity by acquiring the large signal value in the second measurement step A6, which will be described below.

Note that, in the second reaction step, the secondary reactant can also be formed by causing the secondary substance to bind to the detection target in the primary reactant.

As one example in which the second reaction step as the one aspect of the signal amplification step A5 is performed, a description will hereinafter be made on an example in which the detection target is the antigen, the surface acoustic wave element is used as the detection body, a primary antibody is used as the primary substance, the buffer solution is used as the first liquid, and the labeled secondary antibody is used as the secondary substance.

That is, one example of the first embodiment is a detection method of an antigen contained in a sample, may including:
a preparation step of preparing a primary antibody against the antigen, the primary antibody binding to a surface of a surface acoustic wave element;
a first reaction step of supplying the sample to the surface of the surface acoustic wave element, causing the sample to react with the primary antibody against the antigen, and forming a primary composite body of the antigen contained in the sample and the primary antibody on the surface of the surface acoustic wave element;
a first supply step of supplying a buffer solution to the surface of the surface acoustic wave element after the first reaction step;
a first measurement step of measuring a first signal value after the first supply step, the first signal value being based on a surface state of the surface acoustic wave element;
a second reaction step of supplying a labeled secondary antibody against the antigen to the surface of the surface acoustic wave element and forming a secondary composite body of the primary composite body and the labeled secondary antibody on the surface of the surface acoustic wave element after the first measurement step;
a second measurement step of measuring a second signal value after the second reaction step, the second signal value being based on the surface state of the surface acoustic wave element; and
a first detection step of acquiring a detection value from the first signal value which is measured in the first measurement step and the second signal value which is measured in the second measurement step.

### (Second Embodiment)

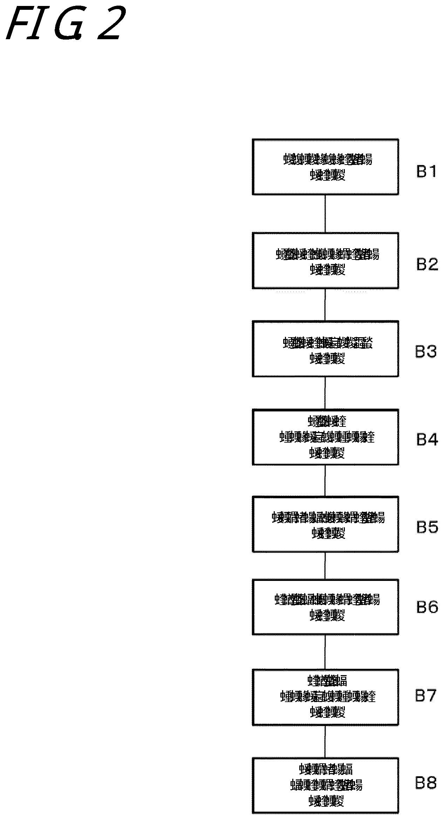

FIG. 2 is a flowchart of a detection method according to a second embodiment of the invention.

The detection method according to the second embodiment of the invention is a detection method of a detection target contained in a sample, including: a preparation step B1 of preparing a primary substance which binds to a surface of the detection body and reacts with the detection target;
a first reaction step B2 of supplying the sample to the surface of the detection body and forming a primary reactant on the surface of the detection body through a reaction of the detection target with the primary substance;
a first supply step B3 of supplying a first liquid to the surface of the detection body after the first reaction step B2;
a first measurement step B4 of measuring a signal value after the first supply step B3, the signal value being based on a surface state of the detection body;
a second reaction step B5 of supplying a secondary substance which reacts with the primary reactant to the surface of the detection body and forming the secondary reactant on the surface of the detection body through a reaction of the primary reactant with the secondary substance after the first measurement step B4;
a third reaction step B6 of supplying a tertiary substance which reacts with the secondary reactant to the surface of the detection body and forming a tertiary reactant on the surface of the detection body through a reaction of the secondary reactant with the tertiary substance after the second reaction step B5;
a third measurement step B7 of measuring a signal value after the third reaction step B6, the signal value being based on the surface state of the detection body; and
a second detection step B8 of acquiring a detection value by subtracting the signal value measured in the first measurement step B4 from the signal value measured in the third measurement step B7.

In this embodiment, the first reaction step B2, the first supply step B3, and the first measurement step B4 are the same as those in the first embodiment. Thus, the description thereon will be omitted.

The second reaction step B5 is performed by using the detection body in the first reaction step B2, the supply channel through which the secondary substance is supplied to this detection body, the pump, and the like. However, a configuration thereof is not limited.

The third reaction step B6 is performed by using the detection body in the second reaction step B5, a supply channel through which the tertiary substance is supplied to this detection body, a pump, and the like. However, a configuration thereof is not limited.

The third measurement step B7 may be performed by using the device or the like which includes the element which inputs the signal to the detection body and which acquires the prescribed signal value on the basis of the signal outputted from the detection body. However, a configuration thereof is not limited, and the third measurement step B7 may be performed in a similar manner to the first measurement step B4.

The second detection step B8 may be performed by using the arithmetic unit or the like which includes the arithmetic element which acquires the detection value from the signal value measured in the first measurement step B4 and the signal value measured in the third measurement step B7. However, a configuration thereof is not limited.

In this embodiment, the second reaction step B5 is performed as an aspect of the additional reaction step, and, after the second reaction step B5, the third reaction step B6 is performed as an aspect of the signal amplification step. In the third reaction step B6, the tertiary substance which reacts with the secondary reactant is supplied to the surface of the detection body, and the tertiary reactant is formed on the surface of the detection body through the reaction of the secondary reactant with the tertiary substance. According to this, by using the tertiary substance, it is possible to amplify the signal value which is derived from the secondary substance, and therefore it is possible to detect the detection target contained in the sample with the higher sensitivity.

In this specification, the "tertiary substance" is not particularly limited as long as the "tertiary substance" is a substance which specifically reacts with the secondary substance. For example, in the case where the secondary substance is the labeled secondary antibody which is labeled with biotin, the "tertiary substance" includes a label detection reagent such as streptavidin which specifically reacts with the label.

Here, as in the above-described example, in the case where the foreign substance which is composed of the different substance from the detection target is further contained in the sample, even when the first liquid is supplied to the surface of the detection body in the first supply step B3, the foreign substance cannot be possibly removed completely from the surface of the detection body. In such a case, the signal value measured in the first measurement step B4 is influenced by the foreign substance remaining on the surface of the detection body. Accordingly, in order to reduce the influence of the foreign substance remaining on the surface of the detection body, the detection value is acquired from the signal value measured in the first measurement step B4 and the signal value measured in the third measurement step B7. In this way, it is possible to reduce the signal value which is derived from the foreign substance remaining on the surface of the detection body, from the signal value measured in the third measurement step B7. This is because a difference between the signal value measured in the first measurement step B4 and the signal value measured in the third measurement step B7 is not substantially influenced by the amount of the foreign substance remaining on the surface of the detection body.

In addition, in the case where the detection target in each of the plural samples is detected, and the viscosity and the density differ among the plural samples, even when the first liquid is supplied to the surface of the detection body in the first supply step B3, the foreign substance which differs from the detection target cannot be removed completely from the surface of the detection body, and therefore the different amount of the foreign substance possibly remains on the surface of the detection body in regard to each of the samples, and the signal value possibly differs among the samples. However, the foreign substance remaining on the surface does not substantially influence the difference between the signal value measured in the first measurement step B4 and the signal value measured in the third measurement step B7, and therefore, by acquiring the detection value from the signal value measured in the first measurement step B4 and the signal value measured in the third measurement step B7, it is possible to reduce the influence of the residue of the foreign substance among the samples.

Just as described, when the influence of the residue of the foreign substance among the samples is reduced, it is possible to further accurately detect the detection target contained in the samples.

Note that, in this embodiment, the secondary substance can be labeled with biotin, and the tertiary substance can contain streptavidin. In this way, it is possible to amplify the signal value which is derived from the secondary substance, and therefore it is possible to detect the detection target with the higher sensitivity.

Note that, in this embodiment, between the second reaction step B5 and the third reaction step B6, the detection method may further comprise a second supply step of supplying the second liquid to the surface of the detection body. In this way, it is possible to remove the secondary substance which has not reacted with the primary reactant in the second reaction step B5, and therefore it is possible to remove an influence of the secondary substance which has not reacted with the primary reactant, from the steps including the third reaction step B6 onward, and thus it is possible to improve the accuracy of the signal value measured in the third measurement step B7.

In this embodiment, between the second reaction step B5 and the second supply step, the detection method may further comprise an intermediate measurement step of measuring the signal value based on the surface state of the detection body. In this way, it is possible to remove the secondary substance which has not reacted with the primary reactant in the second reaction step B5, and therefore it is possible to remove the influence of the secondary substance which has not reacted with the primary reactant, and thus it is possible to improve the accuracy of the signal value measured in the intermediate measurement step.

Note that, in this embodiment, the detection method may further comprise a third detection step of acquiring the detection value from the signal value measured in the intermediate measurement step and the signal value measured in the third measurement step B7. In this way, it is possible to measure the signal value generated in the third reaction step B6.

In this embodiment, between the third reaction step B6 and the third measurement step B7, the detection method may further comprise a third supply step of supplying the third liquid to the surface of the detection body. In this way, it is possible to remove the tertiary substance which has not reacted with the secondary reactant in the third reaction step B6, and therefore it is possible to remove an influence of the tertiary substance which has not reacted with the secondary reactant, from the signal value measured in the third measurement step B7, and it is possible to improve the accuracy of the signal value measured in the third measurement step B7.

In this specification, the "third liquid" may be the buffer solution or the like, for example. Examples of the buffer solution include the phosphate buffer solution; however, the buffer solution is not limited thereto.

Note that, in this embodiment, the first liquid, the second liquid, and the third liquid can be the same type of the liquid.

As one example in which the second reaction step B5 is performed as the one aspect of the additional reaction step and the third reaction step B6 is performed as the one aspect of the signal amplification step, a description will hereinafter be made on an example in which the detection target is the antigen, the surface acoustic wave element is used as the detection body, the primary antibody is used as the primary substance, the buffer solution is used as the first liquid, the labeled secondary antibody is used as the secondary substance, and the label detection reagent is used as the tertiary substance.

That is, one example of a third embodiment may be a detection method of an antigen contained in a sample, including:
a preparation step of preparing a primary antibody against an antigen, the primary antibody binds to a surface of the surface acoustic wave element;
a first reaction step of supplying the sample to the surface of the surface acoustic wave element, causing the sample to react with the primary antibody against the antigen, and forming a primary composite body of the antigen contained in the sample and the primary antibody on the surface of the surface acoustic wave element;
a first supply step of supplying a buffer solution to the surface of the surface acoustic wave element after the first reaction step;
a first measurement step of measuring a signal value after the first supply step, the signal value being based on a surface state of the surface acoustic wave element;
a second reaction step of supplying a labeled secondary antibody against the antigen to the surface of the surface acoustic wave element and forming a secondary composite body of the primary composite body and the labeled secondary antibody on the surface of the surface acoustic wave element after the first measurement step;
a third reaction step of supplying a label detection reagent against the labeled secondary antibody to the surface of the surface acoustic wave element and forming a tertiary composite body of the secondary composite body and the label detection reagent after the second reaction step;
a third measurement step of measuring a signal value after the third reaction step, the signal value being based on the surface state of the surface acoustic wave element; and
a second detection step of acquiring a detection value from the signal value measured in the first measurement step and the signal value measured in the third measurement step.

### <Detection device>

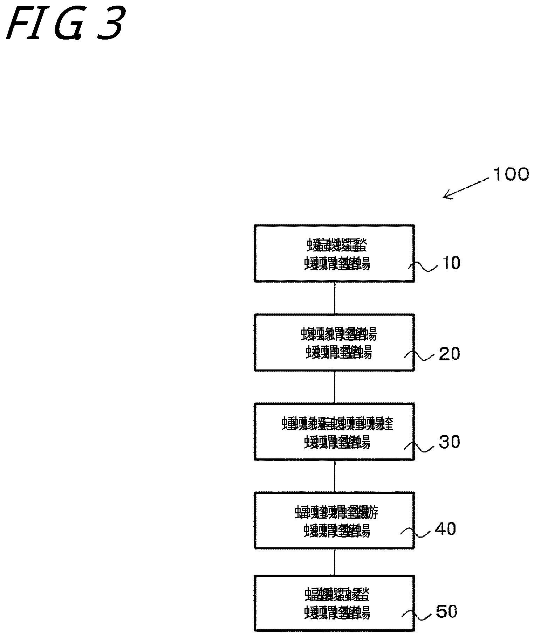

Next, a description will be made on an example of a detection device 100. FIG. 3 is a block diagram of the one example of the detection device. The detection device 100 is configured by connecting a measurement section 30 which measures a signal value, a detecting section 40 which is an arithmetic unit for an arithmetic operation, and a display section 50 which is a display for displaying a detection result to a biosensor device which includes: a supply section 10 which supplies various types of liquids; and a reaction section 20 which performs a precursor reaction and an additional reaction in addition to a reaction for signal amplification.

In the detection device 100 of this embodiment, the supply steps such as the first supply step and the second supply step described above are performed by repeatedly using one supply section 10. Similarly, the reaction steps such as the first reaction step and the second reaction step described above are also performed by repeatedly using one reaction section 20. The above-described measurement steps are performed by repeatedly using one measurement section 30, and the above-described detection steps are performed by repeatedly using one detecting section 40. The display section 50 is not an essential component in this embodiment and only has to be configured to be able to output the detection result from the detecting section 40 to the outside. Electrical connection between the measurement section 30 and the detecting section 40, between the detecting section 40 and the display section 50, and the like may be wired connection using signal cable or the like, or may be wireless connection using an antenna or the like.

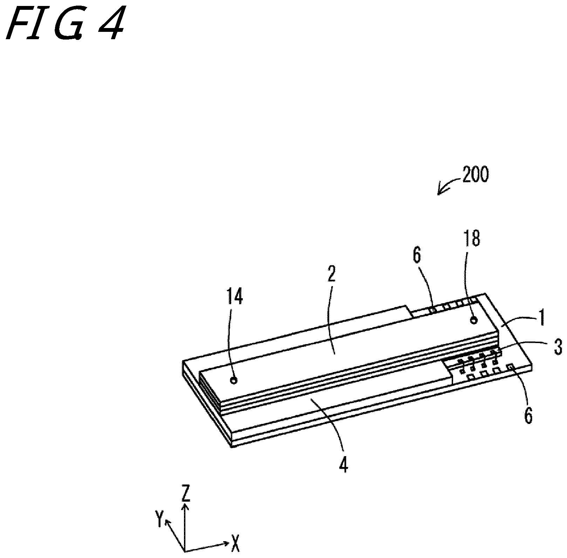

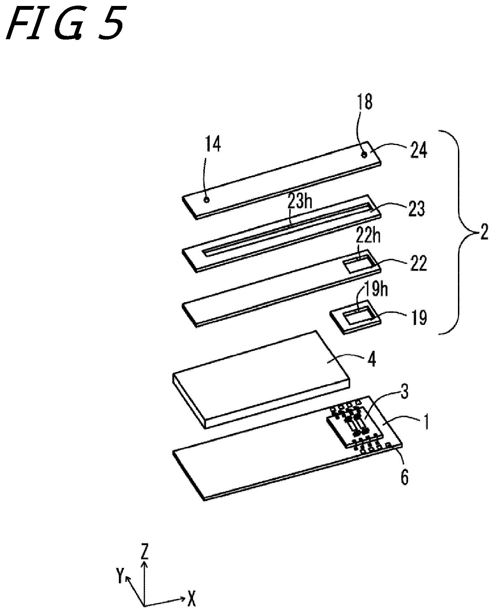

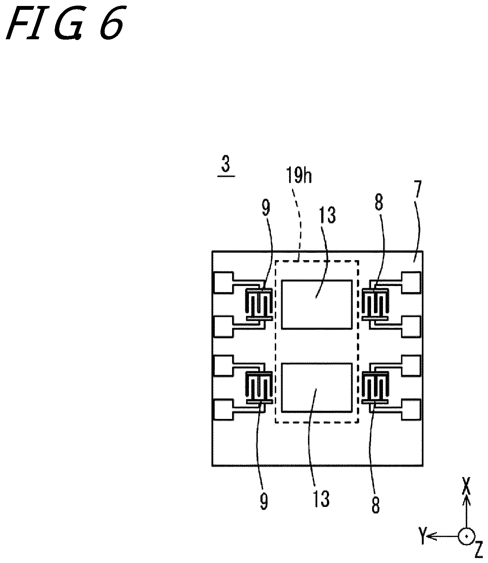

FIG. 4 is a perspective view of a biosensor device 200, FIG. 5 is an exploded perspective view of the biosensor device 200, and FIG. 6 is a plan view of a detecting element 3.

The biosensor device 200 includes a substrate 1, a channel constituent 2, and the detecting element 3. As depicted in FIG. 4, the channel constituent 2 is arranged on the substrate 1 via the detecting element 3 and a support member 4. The channel constituent 2 has an inlet 14 as an entry of a liquid sample on one longitudinal end side, and a channel which is in communication with the inlet 14 is formed therein. The substrate 1 has a plate shape, is a resin substrate, a ceramic substrate, or the like, for example, and is provided with a wiring conductor and the like on a surface layer or an inner layer.

The detecting element 3 is mounted on one end side of an upper surface of the substrate 1. Terminals 6 electrically connected to the detecting element 3 are provided on both sides of the detecting element 3. The device, the arithmetic unit, and the like are connected to the terminals 6.

The detecting element 3 is a surface acoustic wave element and includes a piezoelectric substrate 7, a first IDT (Inter Digital Transducer) electrode 8, a second IDT electrode 9, and a detecting section 13. The piezoelectric substrate 7 is a substrate made of a single crystal with piezoelectricity such as lithium tantalate. The first IDT electrode 8 has a pair of comb electrodes. Each of the comb electrodes has: two bus bars opposed to each other; and plural electrode fingers, each of which extends from one of the bus bars toward the opposed other of the bus bars. The pair of comb electrodes is arranged so that the plural electrode fingers mesh with each other. The second IDT electrode 9 is configured in a similar manner to the first IDT electrode 8. The first IDT electrode 8 and the second IDT electrode 9 constitute IDT electrodes of a transversal type.

The first IDT electrode 8 generates specified surface acoustic wave, and the second IDT electrode 9 receives the SAW generated in the first IDT electrode 8. Each of the first IDT electrode 8 and the second IDT electrode 9 is made of aluminum, an alloy of aluminum and copper, or the like, for example.

The detecting section 13 is provided between the first IDT electrode 8 and the second IDT electrode 9. The detecting section 13 has a double-layered structure composed of chromium and a gold film formed on chromium, for example. The primary substance which reacts with the detection target binds to a surface of a metal film of the detecting section 13. When the sample is supplied to the detecting section, the detection target in the sample reacts with the primary substance, and the primary reactant is thereby formed.

When the first IDT electrode 8, the second IDT electrode 9, and the detecting section 13 are set as a unit, two units thereof are provided in the detecting element 3. For example, one of the detecting sections 13 can measure the sample while the other detecting section 13 can measure a reference value. For example, the primary substance which reacts with the detection target does not bind to the other detecting section 13.

In such a detecting element 3 using the SAW, a signal at a specified voltage is first applied to the first IDT electrode 8 from the outside. In the first IDT electrode 8, a surface of the piezoelectric substrate 7 is excited, and a SAW at a specified frequency is generated. The generated SAW is partially propagated toward the detecting section 13, passes through the detecting section 13, and is received by the second IDT electrode 9. In the detecting section 13, the primary reactant is formed in accordance with the amount of the detection target, and the mass of the detecting section 13 is increased by the amount of the primary reactant. When a phase of the SAW which passes through the detecting section 13 varies in conjunction with an increase in the mass, the voltage corresponding to the variation is generated in the second IDT electrode 9. Then, a difference between a phase of the signal applied to the first IDT electrode 8 and a phase of the signal outputted from the second IDT electrode 9 is measured as a phase variation.

The support member 4 is further mounted on the upper surface of the substrate 1, and the support member 4 supports the channel constituent 2. The channel constituent 2 is arranged to cover at least a part of the detecting element 3. For example, the channel constituent 2 includes a first adhesion layer 19, a first hydrophilic sheet 22, a second adhesion layer 23, and a second hydrophilic sheet 24.

The first adhesion layer 19 is a frame body provided with a through hole 19h, and the detecting element 3 is partially exposed through the through hole 19h. The first hydrophilic sheet 22 is laminated on the first adhesion layer 19. The first hydrophilic sheet 22 is provided with a through hole 22h which is similar to the through hole 19h, and the first adhesion layer 19 and the first hydrophilic sheet 22 are laminated so that the through holes are in communication with each other. The second adhesion layer 23 is laminated on the first hydrophilic sheet 22. The second adhesion layer 23 is provided with a through hole 23h which constitutes the channel and extends longitudinally. The through hole 23h extends to such a position that one end thereof overlaps the through hole 22h. The second hydrophilic sheet 24 is laminated on the second adhesion layer 23. The inlet 14 and an outlet 18, each of which is formed as a through hole, are provided near respective ends of the second hydrophilic sheet 24. The inlet 14 and the outlet 18 are formed at overlapping positions with the through hole 23h.

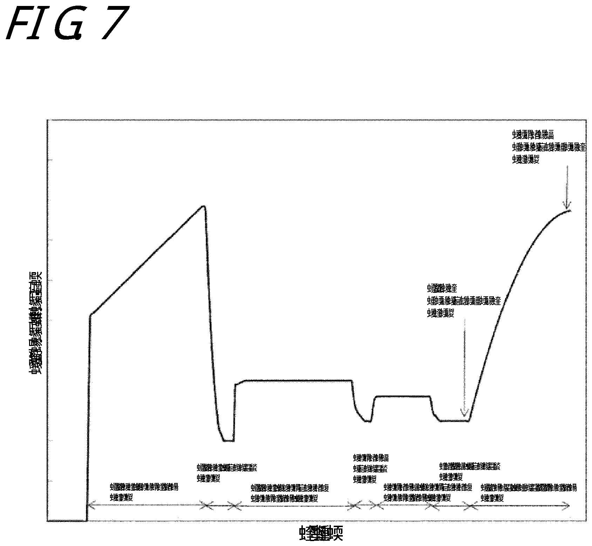

A schematic graph in FIG. 7 illustrates an example of the signal value which is acquired when the precursor reaction step is performed by using the biotin-labeled secondary antibody and alkaline phosphatase-labeled streptavidin as the precursor reaction substances, the signal amplification step is performed by using 5-bromo-4-chloro-3-indolylphosphate/nitro blue tetrazolium as the signal amplification substance, and the signal value is acquired from a SAW element.

A description will be made on the steps performed in the schematic graph depicted in FIG. 7. First, the first reaction step and the first supply step were performed to form the primary reactant. Next, a first precursor reaction step was performed to cause the biotin-labeled secondary antibody to react with the primary reactant. After the first precursor reaction step, the second supply step of supplying 10 mM PBS (10 mM Phosphate, 137 mM Sodium Chloride, 2.7 mM Potassium Chloride, 0.005% Tween 20 (registered trademark), pH 7.4) was performed to remove the free biotin-labeled secondary antibody. After the second supply step, a second precursor reaction step was performed to cause the reaction of alkaline phosphatase-labeled streptavidin. After the second precursor reaction step, the third supply step of supplying 10 mM PBS (10 mM Phosphate, 137 mM Sodium Chloride, 2.7 mM Potassium Chloride, 0.005% Tween 20 (registered trademark), pH 7.4) was performed to remove free alkaline phosphatase-labeled streptavidin. After the third supply step, the first measurement step was performed to acquire the first signal value. After the first measurement step, the signal amplification step was performed to cause the reaction of 5-bromo-4-chloro-3-indolylphosphate/nitro blue tetrazolium. After the signal amplification step, the second measurement step was performed, and the second signal value was acquired. Finally, the detection value was acquired by subtracting the first signal value from the second signal value.

### Example

A description will hereinafter be made on an example of the detection method according to the above-described embodiment.

In this example, the biological sample was used, and the detection target contained in the biological sample was detected.

As the biological samples, two different types of the sample, each of which contained the same amount of the antigen, (biological samples A, B) were prepared. That is, although viscosity and contained substances differed, the biological sample A and the biological sample B contained the same amount of the antigen.

The surface acoustic wave element (the SAW element) was used as the detection body, and the SAW element, to a surface of which the antibody as the primary substance (hereinafter simply referred to as the "antibody") was boned in advance, was prepared. Here, the antibody is a substance binding to the detection target.

As Example 1, first, as the first reaction step, the biological sample was supplied to the surface of the SAW element, and the antigen as the detection target (hereinafter simply referred to as the "antigen") reacted with the antibody to form the primary reactant.

Next, in the first supply step, the buffer solution of 10 mM PBS (10 mM Phosphate, 137 mM Sodium Chloride, 2.7 mM Potassium Chloride, 1 mM MgCl₂, 0.005% Tween 20 (registered trademark), pH 7.4) as the first liquid was supplied to the surface of the SAW element.

Next, in the first measurement step, the phase variation between the input signal and the output signal in the SAW element was acquired as the signal value.

Next, in the signal amplification step, a biotin-modified secondary antibody as the signal amplification substance was supplied to the surface of the SAW element, and the primary reactant reacted with the biotin-modified secondary antibody to form a composite body of the primary reactant and the biotin-modified secondary antibody.

Next, in the second measurement step, the phase variation between the input signal and the output signal in the SAW element was acquired as the signal value.

Next, in the first detection step, the detection value was acquired by subtracting the first signal value that was measured in the first measurement step from the second signal value that was measured in the second measurement step.

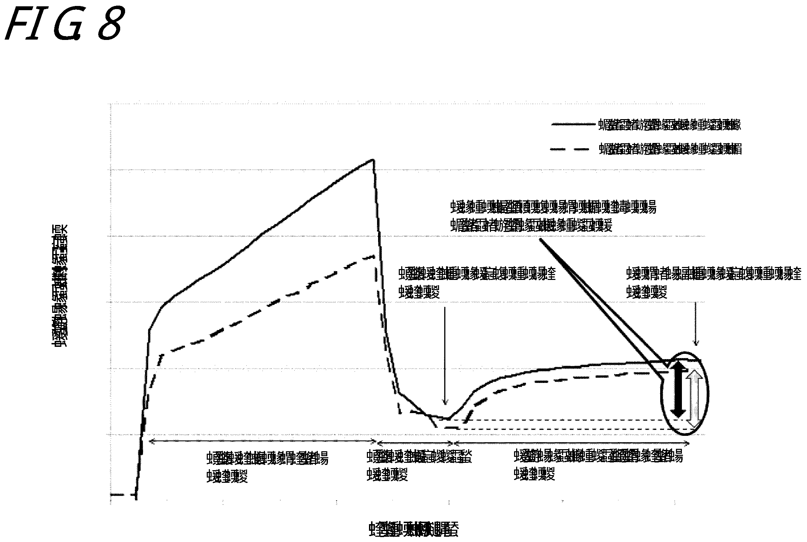

The signal value for each of the biological samples which was acquired as described above is depicted in a graph as in FIG. 8.

As depicted in FIG. 8, in Example 1, the biological sample A and the biological sample B contained the same amount of the antigen, and the detection value of the biological sample A and the detection value of the biological sample B were the same.

From the above, according to the detection method according to the embodiment of the invention, it was concluded that the influence of the difference between the biological samples was reduced and that the detection target (the antigen) contained in each of the biological samples could accurately be detected.

Meanwhile, as a comparative example, the first measurement step as described above was not performed, and a value of the phase variation measured in the second measurement step was acquired as the detection value.

In such a comparative example, it was concluded that the detection value of the biological sample A and the detection value of the biological sample B differed from each other and that the detection target (the antigen) contained in each of the biological samples could not be accurately detected due to the influence of the difference between the biological samples (see FIG. 8), the biological sample A and the biological sample B containing the same amount of the antigen.

Note that, as Example 2, the biological sample was not used, the sample that was acquired by diluting the same amount of the antigen as that in Example 1 by the buffer solution of 10 mM PBS (10 mM Phosphate, 137 mM Sodium Chloride, 2.7 mM Potassium Chloride, 1 mM MgCl₂, 0.005% Tween 20 (registered trademark), pH 7.4) was used, and the detection value that was acquired in the same method as in Example 1 was the same as that in Example 1.

From the above, it was proved that the detection target (the antigen) contained in the biological sample could be accurately detected in Example 1.

The invention is not limited to the above examples, and exhibits the same effect even when the detection target is a different type of the antigen.

It is needless to say that the invention is not limited to the configuration described in each of the above-described embodiments and modified examples, and improvements and modifications are possible without departing from the scope of the invention.

### Reference Signs List

1: Substrate
2: Channel constituent
3: Detecting element
4: Support member
6: Terminal
7: Piezoelectric substrate
8: First IDT electrode
9: Second IDT electrode
10: Supply section
13: Detecting section
14: Inlet
20: Reaction section
30: Measurement section
40: Detecting section
50: Display section
100: Detection device
200: Biosensor device

## Claims

1. A detection method of a detection target contained in a sample, comprising:
a preparation step of preparing a primary substance which binds to a surface of a detection body and reacts with the detection target;
a first reaction step of supplying the sample to the surface of the detection body and forming a primary reactant on the surface of the detection body through a reaction of the detection target with the primary substance;
a first supply step of supplying a first liquid to the surface of the detection body after the first reaction step;
a first measurement step of measuring a first signal value after the first supply step, the first signal value being based on a surface state of the detection body;
a signal amplification step of supplying a signal amplification substance to the surface of the detection body and changing the surface state of the detection body through a reaction in which the primary reactant formed in the first reaction step is involved, after the first measurement step;
a second measurement step of measuring a second signal value after the signal amplification step, the second signal value being based on the surface state of the detection body; and
a first detection step of acquiring a detection value from the first signal value and the second signal value.

2. The detection method according to claim 1, further comprising:
a second supply step of supplying a second liquid to the surface of the detection body, between the signal amplification step and the second measurement step.

3. The detection method according to claim 1 or 2, further comprising:
an additional reaction step of supplying at least one additional reaction substance to the surface of the detection body, between the first measurement step and the signal amplification step.

4. The detection method according to claim 3, wherein the at least one additional reaction substance has a plurality of additional reaction substances, and the plurality of additional reaction substances are supplied one by one.

5. The detection method according to claim 1 or 2, further comprising:
a precursor reaction step of supplying at least one precursor reaction substance to the surface of the detection body, between the first reaction step and the first supply step.

6. The detection method according to claim 5, wherein the at least one precursor reaction substance has a plurality of precursor reaction substances, and the plurality of precursor reaction substances are supplied one by one.

7. The detection method according to any one of claims 1 to 6, wherein
the sample further contains a foreign substance which differs from the detection target, and
the foreign substance is adhered to the surface of the detection body in the first reaction step, and the foreign substance remains on the surface of the detection body after the first supply step.

8. The detection method according to any one of claims 1 to 7, wherein the sample is a biological sample.

9. The detection method according to any one of claims 1 to 8, wherein in the first reaction step, the primary reactant is formed by causing the detection target to bind to the primary substance.

10. The detection method according to any one of claims 1 to 8, wherein in the first reaction step, the primary reactant is formed by causing the detection target to bind to a part of the primary substance and dissociating the part from the primary substance.

11. The detection method according to any one of claims 1 to 10, wherein
a surface acoustic wave element is formed in the surface of the detection body, and
a signal value based on the surface state of the detection body is a phase characteristic value of the surface acoustic wave element.

12. The detection method according to any one of claims 1 to 10, wherein a signal value based on the surface state of the detection body is a value measured by a method selected from a QCM (Quartz Crystal Microbalance) sensor, a SPR (Surface Plasmon Resonance) sensor, and a FET (Field Effect Transistor) sensor.

13. The detection method according to any one of claims 1 to 12, wherein the second signal value is larger than the first signal value.

14. The detection method according to any one of claims 1 to 13, wherein the first liquid is a buffer solution.

15. The detection method according to any one of claims 2 to 14, wherein the first liquid and the second liquid are the same.

16. A device for detecting a detection target contained in a sample, comprising:
a first reaction section which supplies the sample to a primary substance and forms a primary reactant on a surface of a detection body through a reaction of the detection target with the primary substance, the primary substance binding to the surface of the detection body and reacting with the detection target;
a first supply section which supplies a first liquid to the surface of the detection body;
a first measurement section which measures a first signal value which is based on a surface state of the detection body, after the first supply section supplies the first liquid to the surface of the detection body;
a signal amplification section which supplies a signal amplification substance to the surface of the detection body and changes the surface state of the detection body through a reaction in which the primary reactant is involved;
a second measurement section which measures a second signal value which is based on the surface state of the detection body to which the signal amplification substance has been supplied;
a first detecting section which acquires a detection value from the first signal value and the second signal value; and
a display section which displays the detection value acquired by the first detecting section.
